# EUROPEAN PATENT APPLICATION

(11) **EP 3 557 232 A1**
(43) Date of publication of application: **23.10.2019**
(21) Application number: 18020261.6
(22) Date of filing: 18.06.2018
(51) Int. Cl.: G01N 21/952, G01N 33/483, G03B 17/56

(54) **ANALYZER OF SEQUENTIAL DEFORMATION OF BLOOD VESSELS' WALL**

(30) Priority: 17.04.2018 PL 42525418
(71) Applicant: Politechnika Lodzka, 90-924 Lodz (PL)
(72) Inventor: Polanczyk, Andrzej, 94-047 Lodz (PL); Polanczyk, Maciej, 94-047 Lodz (PL); Piechota-Polanczyk, Aleksandra, 94-004 Lodz (PL); Podgorski, Michal, 91-855 Lodz (PL)

(57) **Abstract**

Analyzer of sequential deformation of a blood vessels' walls is a horizontally localized circular ring (1) with through, threaded holes (2) of equal diameter arranged on the circle having the radius greater than the inner radius of the circular ring (1) and smaller than the external radius of the circular ring (1) in equal distance between each two holes (2). To the upper surface of the circular ring (1) flat bars (5), in equal distance between each two flat bars (5), are attached, each of a flat bar (5) is equipped with through, threaded holes (7) having the diameter equal with the diameter of holes (2) in the circular ring (1), holes (7) are arranged along the longitudinal axis of the flat bar (5) with equal distance between each two holes and both ends of the flat bar (5), furthermore in one of the holes (7) in each of the flat bar (5) one end of a vertically located stanchion (6) is screwed and secured at the bottom part of the circular ring (1) with a positioning nut (4), while in one of the remaining holes (7) of each flat bar (5) localized above the hole (2) in the circular ring (1) as well as in the hole (2) in the circular ring (1) a screw joint (3) combining flat bar (5) and the circular ring (1) is positioned, moreover on the other end of vertically located stanchion (6) of each flat bar (5) a digital camera (8) connected to the image acquisition device is attached.

## Description

The invention relates to an analyzer of sequential deformation of an artificial and a real blood vessels' wall in *ex-vivo* conditions.

So far hemodynamics of blood flow in human vessels for diagnosis of pathological changes was analyzed with the use of USG-Doppler. This technique allows analysis of blood hemodynamic in vessels located in human organism. However, it does not allow to analyze vessel's wall deformations. Taking into consideration that with analysis of vessel's wall deformation one can determine the changes in wall structure, which potentially may lead to pathophysiological changes of the vessel's wall or implanted artificial structures such as stent-grafts, it is an important parameter that should be analyzed together with the character of blood flow. Moreover, human vessel is a biological material which in *ex-vivo* conditions, that simulates human organism, should be investigated as fast as possible so that its mechanical and biological properties were as closes as possible to those in its natural environment. Therefore, wall deformation analysis should be conducted in the real time, which allows immediate analysis of gathered results. Furthermore, for a reliable analysis of blood vessel's wall deformation the analysis should be conducted in the conditions simulating a working heart i.e. analysis of many sequential deformations of vessel's wall. Additionally, the possibility to analyze the deformation of the blood vessel wall under ex-vivo conditions is important because it is not possible to analyze any hemodynamics of blood flow in the human body, as an extremely high or low flow rates could involve the risk of of patient death. Therefore, it is crucial to analyze wall deformation in *ex-vivo* conditions. So far it is not possible to analyze wall deformation in the real time in *ex-vivo* conditions. Also, it is not possible to analyze wall deformation with the USG-Doppler technique. Therefore, there is a need to develop an analyzer of sequential deformations of blood vessels' wall in real time in *ex-vivo* conditions.

This problem is solved by the device according to the invention.

Analyzer of sequential deformation of a blood vessels' walls according to the invention is a circular ring localized horizontally with through, threaded holes of equal diameter,, arranged on the circle having radius greater than the inner radius of the circular ring and smaller than the external radius of the circular ring, in equal distance between each two holes. To the upper surface of a circular ring, flat bars, in equal distance between each two flat bars, are attached Each of a flat bar is equipped with through, threaded holes having the diameter equal with the diameter of holes in the circular ring The holes are arranged along the longitudinal axis of the flat bar with equal distance between each two holes and both ends of the flat bar. In one of the holes of each flat bar the one end of a vertically located stanchion is screwed and secured at the bottom part of the circular ring with a positioning nut. While, in one of the remaining holes, of each flat bar, localized above the hole in circular ring as well as in the hole in the circular ring a screw joint combining flat bar and the circular ring is positioned. The other end of a vertically located stanchion of each flat bar, a digital camera connected to the image acquisition device is localized. The circular ring, flat bars and stanchions are made of aluminum alloy or acid resistant steel. While, screw joints are made of acid resistant steel. The circular ring of the analyzer preferably comprises thirty six through, threaded holes, with the distance between each two holes equal to 10°.. Analyzer preferably comprises here nine flat bars attached to the upper surface of the circular ring, with the distance between each two flat bars equal to 40°. Each of the flat bar attached to the upper surface of the circular ring comprises three through, threaded holes, arranged in distances equal to ¼ length of flat bar, between each two holes and from the ends of the flat bar.

The analyzer according to the invention provides recording of wall deformation for any type of blood vessel, simultaneously on whole blood vessel's perimeter, during the reconstruction of any blood hemodynamics parameters in *ex-vivo* conditions. Thereby, analyzer enables analysis of mechanical properties of different spatial configuration of any type of blood vessel.

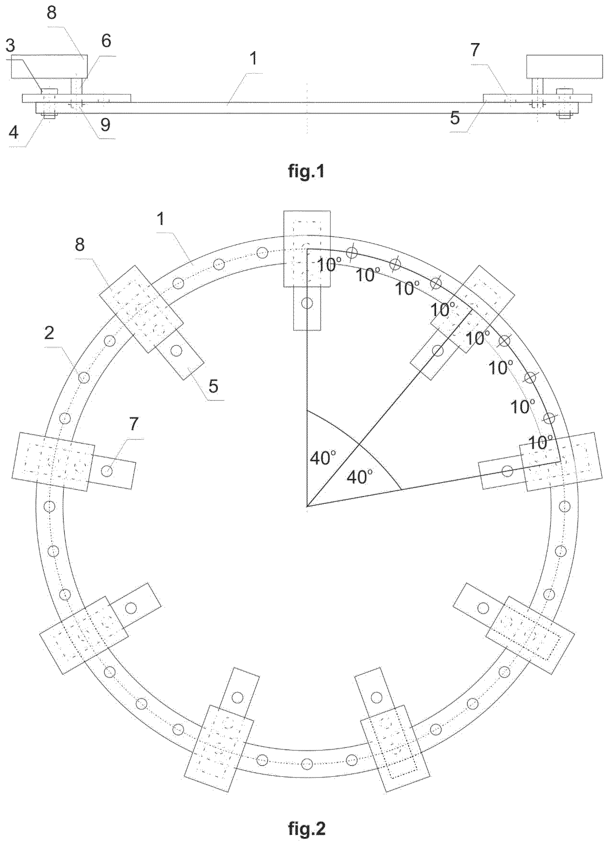

The subject of the invention was presented in embodiment on the drawing on which fig.1 provides a side view and fig.2 provides a top view.

Analyzer of sequential deformation of a blood vessels' walls is a circular ring 1 localized horizontally with, thirty six through, threaded holes 2 of equal diameter arranged on the circle having the radius greater than the inner radius of the circular ring 1 and smaller than the external radius of the circular ring 1, with the distance between each two holes equal to 10°. To the upper surface of a circular ring 1, nine flat bars 5 are attached, with the distance between each two flat bars 5 equal to 40°. Each flat bar 5, comprises three through, threaded holes 7 of diameter equal to the diameter of the holes 2 in the circular ring 1, and are arranged along the longitudinal axis of the flat bar 5, in a distance equal to ¼ length of flat bar, between each two holes 7 as well as from both ends of the flat bar 5. In the first extreme hole 7 of each flat bar 5 from the middle of circular ring 1 a vertically located cylindrical stanchion 6 is screwed and secured at the bottom part of the circular ring 1 with the positioning nut 4. In the second extreme hole 7 of each flat bar 5 localized above the hole 2 in circular ring 1 as well as in a hole 2 localized in the circular ring 1, a screw 3 combining flat bar 5 and circular ring 1 is screwed and secured at the bottom part of the circular ring 1 with positioning nut 9. On the other side of vertically located screwed stanchion 6 of each flat bar 5 a digital camera 8 connected to the image acquisition device is localized. The circular ring 1, flat bars 5 and stanchions 6 are made of aluminum alloy or acid resistant steel. While, screw joints 3 and positioning nuts 4, 9 are made of acid resistant steel.

Each time the analyzed vessel is placed inside a circular ring 1 and with the use of digital cameras 8 placed on the stanchions 6 localized on the perimeter of circular ring 1 a vessel's wall deformation in three dimensional configuration is simultaneously monitored with nine cameras 8. Simultaneously movement of flat bars 5 in the same direction on the perimeter of circular ring 1 with the use of threaded holes 2, with the distance between each two holes 2 equal to 10°, allows to modify each monitoring position with a constant step equal to 10°. Additionally each flat bar 5 is equipped with several holes 7 that allow movement of cylindrical stanchions 6, on the top of which digital cameras 8 are localized, along the longitudinal axis of the flat bar 5.. This allows to measure vessel's wall deformation for different distances from the middle of circular ring 1. Threaded of the cylindrical stanchions 6 allows displacement of a digital camera 8 in the vertical plane in the axis of threaded holes 7 with the distance equal to the step of thread.

## Claims

1. Analyzer of sequential deformation of a blood vessels' walls, **characterized by**, horizontally localized circular ring (1) with through, threaded holes (2) of equal diameter, arranged on the circle having the radius greater than the inner radius of the circular ring (1) and smaller than the external radius of the circular ring (1) in equal distance between each two holes (2), in addition to the upper surface of the circular ring (1) flat bars (5), in equal distance between each two flat bars (5)are attached, each of a flat bar (5) is equipped with through, threaded holes (7) having the diameter equal with the diameter of holes (2) in the circular ring (1), holes (7) are arranged along the longitudinal axis of the flat bar (5) with equal distance between each two holes and both ends of the flat bar (5), furthermore in one of the holes (7) in each of the flat bar (5) one end of a vertically located stanchion (6) is screwed and secured at the bottom part of the circular ring (1) with a positioning nut (4), while in one of the remaining holes (7) of each flat bar (5) localized above the hole (2) in the circular ring (1) as well as in the hole (2) in the circular ring (1) a screw joint (3) combining flat bar (5) and the circular ring (1) is positioned, moreover on the other end of vertically located stanchion (6) of each flat bar (5) a digital camera (8) connected to the image acquisition device is attached.

2. The analyzer according to the claim 1 is **characterized in that** the circular ring (1) comprises thirty six through, threaded holes (2), with the distance between each two holes (2) equal to 10°.

3. The analyzer according to the claim 1 is **characterized in that** it comprises nine flat bars (5) attached to the upper surface of the circular ring (1) with the distance between each two flat bars (5) equal to 40°.

4. The analyzer according to the claim 1 is **characterized in that**, each of the flat bar (5) attached to the upper surface of the circular ring (1) comprises three through threaded holes (7) arranged in distance equal to ¼ length of flat bar (5), between each two holes (7) and from the ends of the flat bar (5).

5. The analyzer according to the claim 1 is **characterized by**, the circular ring (1), flat bars (5) and stanchions (6) made of aluminum alloy or acid resistant steel, and screw joints (3, 4, 9) elements made of acid resistant steel.
